# EUROPÄISCHE PATENTSCHRIFT

(11) **EP 1 021 417 B1**
(45) Veröffentlichungstag und Bekanntmachung des Hinweises auf die Patenterteilung: **10.03.2004**
(21) Anmeldenummer: 98951358.5
(22) Anmeldetag: 07.09.1998
(51) Int. Cl.: C07D 239/54, C07D 409/12, C07D 401/12, A01N 47/38, A01N 43/54

(54) **SUBSTITUIERTE PHENYLURACILE**
SUBSTITUTED PHENYLURACILS
PHENYLURACILE SUBSTITUE

(30) Priorität: 10.09.1997 DE 19739638
(43) Veröffentlichungstag der Anmeldung: 26.07.2000
(73) Patentinhaber: Bayer CropScience AG, 40789 Monheim (DE)
(72) Erfinder: ANDREE, Roland, D-40764 Langenfeld (DE); SCHALLNER, Otto, D-40789 Monheim (DE); VOIGT, Katharina, D-52080 Aachen (DE); DOLLINGER, Markus, Overland Park, KS 66213 (US); SANTEL, Hans-Joachim, D-51371 Leverkusen (DE)
(86) Internationale Anmeldenummer: PCT/EP1998/005659
(87) Internationale Veröffentlichungsnummer: WO 1999/012913

(56) Entgegenhaltungen:
- EP-A- 0 545 206
- WO-A-95/17096
- WO-A-97/08953
- US-A- 5 169 430
- US-A- 5 344 812
- US-A- 5 399 543
- PATENT ABSTRACTS OF JAPAN vol. 16, no. 485 (C-0993), 8. Oktober 1992 & JP 04 178373 A (NISSAN CHEM IND LTD), 25. Juni 1992 in der Anmeldung erwähnt
- PATENT ABSTRACTS OF JAPAN vol. 17, no. 339 (C-1075), 28. Juni 1993 & JP 05 039272 A (NISSAN CHEM IND LTD), 19. Februar 1993 in der Anmeldung erwähnt
- PATENT ABSTRACTS OF JAPAN vol. 17, no. 644 (C-1134), 30. November 1993 & JP 05 202031 A (NISSAN CHEM IND LTD), 10. August 1993 in der Anmeldung erwähnt
- PATENT ABSTRACTS OF JAPAN vol. 17, no. 309 (C-1070), 14. Juni 1993 & JP 05 025144 A (NISSAN CHEM IND LTD), 2. Februar 1993

## Beschreibung

Die Erfindung betrifft neue substituierte Phenyluracile, Verfahren zu ihrer Herstellung und ihre Verwendung als Herbizide.

Eine große Zahl von substituierten Aryluracilen ist bereits aus der (Patent-)Literatur bekannt (vgl. JP-A-04178373 - zitiert in Chem. Abstracts 118:59721; JP-A-05039272 - zitiert in Chem. Abstracts 119:180805; JP-A-05202031 - zitiert in Chem. Abstracts 120:107048; EP-A-545206; US-A-5169430; US-A-5344812; US-A-5399543; WO-A-95/17096; WO-A-97/08953). Diese Verbindungen haben jedoch bisher keine besondere Bedeutung erlangt.

Es wurden nun neue substituierte Phenyluracile der allgemeinen Formel (I) gefunden, 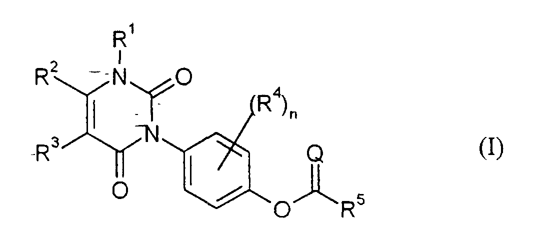 in welcher
- n: für die Zahlen 0, 1, 2 oder 3 steht,
- Q: für Sauerstoff oder Schwefel steht,
- R¹: für Wasserstoff, für Amino oder für gegebenenfalls durch Cyano, Fluor, oder Chlor substituiertes Alkyl mit 1 bis 4 Kohlenstoffatomen steht,
- R²: für Cyano, Carboxy, Carbamoyl, Thiocarbamoyl oder für jeweils gegebenenfalls durch Cyano, Fluor oder Chlor substituiertes Alkyl oder Alkoxycarbonyl mit jeweils bis zu 4 Kohlenstoffatomen steht,
- R³: für Wasserstoff, Fluor, Chlor, Brom oder Alkyl mit 1 bis 4 Kohlenstoffatomen steht,
- R⁴: für Nitro, Amino, Hydroxy, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino, Alkylsulfonylamino, Bis-alkylcarbonyl-amino, Bis-alkylsulfonyl-amino oder N-Alk-ylcarbonyl-N-alkylsulfonyl-amino mit jeweils bis zu 5 Kohlenstoffatomen in den Alkylgruppen steht, und
- R⁵: für Wasserstoff, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 5 Kohlenstoffatomen, für jeweils gegebenenfalls durch Cyano oder Halogen substituiertes Alkenyl, Alkenyloxy, Alkenylamino, Alkinyl, Alkinyloxy oder Alkinylamino mit jeweils bis zu 5 Kohlenstoffatomen, für jeweils gegebenenfalls durch Cyano, Halogen oder C₁-C₄-Alkyl substituiertes Cycloalkyl, Cycloalkyloxy, Cycloalkylamino, Cycloalkylalkyl, Cycloalkylalkoxy oder Cycloalkylalkylamino mit jeweils 3 bis 6 Kohlenstoffatomen in den Cycloalkylgruppen und gegebenenfalls 1 bis 3 Kohlenstoffatomen in den Alkylteilen, für jeweils gegebenenfalls durch Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Phenyl substituiertes Aryl, Aryloxy, Arylthio, Arylamino, Arylalkyl, Arylalkoxy, Arylalkylthio oder Arylalkylamino mit jeweils 6 oder 10 Kohlenstoffatomen in den Arylgruppen und gegebenenfalls 1 bis 3 Kohlenstoffatomen in den Alkylteilen, oder für jeweils gegebenenfalls durch Cyano, Halogen, C₁-C₄-Alkyl, C₁-C₄-Halogenalkyl, C₁-C₄-Alkoxy oder C₁-C₄-Halogenalkoxy substituiertes Heterocyclyl oder Heterocyclylalkyl aus der Reihe Furyl, Tetrahydrofuryl, Furyl-C₁-C₄-alkyl, Benzofuryl, Benzofuryl-C₁-C₄-alkyl, Thienyl, Thienyl-C₁-C₄-alkyl, Benzothienyl, Benzothienyl-C₁-C₄-alkyl, Oxazolyl, Oxazolyl-C₁-C₄-alkyl, Benzoxazolyl, Benzoxazolyl-C₁-C₄-alkyl, Isoxazolyl, Isoxazolyl-C₁-C₄-alkyl, Benzisoxazolyl, Benzisoxazolyl-C₁-C₄-alkyl, Thiazoyl, Thiazolyl-C₁-C₄-alkyl, Benzthiazolyl, Benzthiazolyl-C₁-C₄-alkyl, Pyrazolyl, Pyrazolyl-C₁-C₄-alkyl, Oxadiazolyl, Oxadiazolyl-C₁-C₄-alkyl, Thiadiazolyl, Thiadiazolyl-C₁-C₄-alkyl, Pyridyl, Pyridyl-C₁-C₄-alkyl, Chinolyl, Chinolyl-C₁-C₄-alkyl, Pyrimiyl, Pyrimidyl-C₁-C₄-alkyl steht.

Die Erfindung betrifft insbesondere Verbindungen der Formel (I), in welcher
- n: für die Zahlen 0, 1 oder 2 steht,
- Q: für Sauerstoff oder Schwefel steht,
- R¹: für Wasserstoff, Amino, Methyl oder Ethyl steht,
- R²: für Cyano, Carboxy, Carbamoyl, Thiocarbamoyl, Methyl, Ethyl, Fluormethyl, Chlormethyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Chlorfluorethyl, Trifluorethyl, Trichlorethyl, Chlordifluorethyl, Fluordichlorethyl, Pentafluorethyl, Methoxycarbonyl oder Ethoxycarbonyl steht,
- R³: für Wasserstoff, Chlor, Brom oder Methyl steht,
- R⁴: für Nitro, Amino, Hydroxy, Carboxy, Cyano, Carbamoyl, Thiocarbamoyl, Sulfo, Chlorsulfonyl, Aminosulfonyl, Fluor, Chlor, Brom, oder für jeweils gegebenenfalls durch Cyano, Fluor, Chlor, Brom, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, Methylsulfonyl, Ethylsulfonyl, Methylamino, Ethylamino, n- oder i-Propylamino, n-, i-, s- oder t-Butylamino, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, Acetylamino, Propionylamino, n- oder i-Butyroylamino, Methoxycarbonylamino, Ethoxycarbonylamino, Methylsulfonylamino, Ethylsulfonylamino, n- oder i-Propylsulfonylamino, n-, i-, s- oder t-Butylsulfonylamino, Bis-methylsulfonyl-amino, Bis-ethylsulfonyl-amino, N-Acetyl-N-methylsulfonyl-amino oder N-Acetyl-N-ethylsulfonyl-amino steht, und
- R⁵: für Wasserstoff, für jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino odec Diethylamino, für jeweils gegebenenfalls durch Cyano, Fluor, Chlor oder Brom substituiertes Ethenyl, Propenyl, Butenyl, Propenyloxy, Butenyloxy, Propenylamino, Butenylamino, Ethinyl, Propinyl, Butinyl, Propinyloxy, Butinyloxy, Propinylamino oder Butinylamino, für jeweils gegebenenfalls durch Cyano, Fluor, Chlor, Methyl oder Ethyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylamino, Cyclobutylamino, Cyclo-pentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclopropylmethylamino, Cyclobutylmethylamino, Cyclopentylmethylamino oder Cyclohexylmethylamino, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n-oder i-Propoxy, Difluormethoxy, Trifluormethoxy, Methylthio, Ethylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl oder Phenyl substituiertes Phenyl, Phenoxy, Phenylthio, Phenylamino, Benzyl, Benzyloxy, Benzylthio oder Benzylamino, oder für jeweils gegebenenfalls durch Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl oder Heterocyclylalkyl aus der Reihe Furyl, Tetrahydrofuryl, Furylmethyl, Benzofuryl, Benzofurylmethyl, Thienyl, Thienylmethyl, Benzothienyl, Benzothienylmethyl, Oxazolyl, Oxazolylmethyl, Benzoxazolyl, Benzoxazolylmethyl, Isoxazolyl, Isoxazolylmethyl, Benzisoxazolyl, Benzisoxazolylmethyl, Thiazoyl, Thiazolylmethyl, Benzthiazolyl, Benzthiazolylmethyl, Pyrazolyl, Pyrazolylmethyl, Oxadiazolyl, Oxadiazolylmethyl, Thiadiazolyl, Thiadiazolylmethyl, Pyridyl, Pyridylmethyl, Chinolyl, Chinolylmethyl, Pyrimidyl, Pyrimidylmethyl steht.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Die neuen substituierten Phenyluracile der allgemeinen Formel (I) zeichnen sich durch starke und selektive herbizide Wirksamkeit aus.

Man erhält die neuen substituierten Phenyluracile der allgemeinen Formel (I), wenn man Hydroxyphenyluracile der allgemeinen Formel (II) 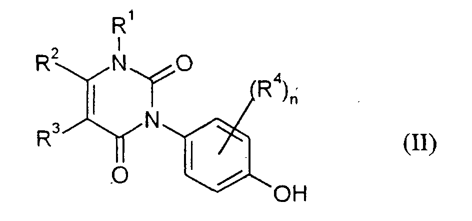 in welcher
- n, R¹, R², R³ und R⁴: die vorstehend angegebene Bedeutung haben
mit Acylierungsmitteln der allgemeinen Formel (III) 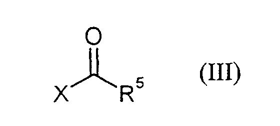 in welcher
- R⁵: die vorstehend angegebene Bedeutung hat und
- X: für Halogen oder die Gruppierung -O-CO-R⁵ steht,
oder für den Fall, daß in der Formel (I) R⁵ für Alhylamino, Alkenylamino, Alkinylamino, Cycloalkylamino, Cycloalkylalkylamino, Arylamino oder Arylalkylamino steht, mit entsprechenden Iso(thio)cyanaten der allgemeinen Formel (IV)

Q=C=N-R⁶ (IV)

in welcher
- Q: die vorstehend angegebene Bedeutung hat und
- R⁶: für jeweils gegebenenfalls substituiertes Alkyl, Alkenyl, Alkinyl, Cycloalkyl, Cycloalkylalkyl, Aryl oder Arylalkyl steht,
gegebenenfalls in Gegenwart eines Verdünnungsmittels und gegebenenfalls in Gegenwart eines Reaktionshilfsmittels umsetzt und gegebenenfalls Folgeumsetzungen im Rahmen der Substituentendefinition nach üblichen Methoden durchführt.

Als Folgeumsetzungen sind in diesem Zusammenhang im wesentlichen Substitutionsreaktionen zu verstehen, bei denen Verbindungen der allgemeinen Formel (I), in welcher R¹ für Wasserstoff steht, durch Aminierungsreaktionen, beispielsweise mit 1-Aminooxy-2,4-dinitro-benzol, in entsprechende Aminouracile umgewandelt werden, oder durch Alkylierungsreaktionen in entsprechende Alkyluracile überführt werden (vgl. die Herstellungsbeispiele).

Verwendet man beispielsweise 3-(3-Fluor-4-hydroxy-phenyl)-1-methyl-6-trifluormethyl-(1H,3H)-pyrimidin-2,4-dion und 3,4-Dichlor-benzoylchlorid als Ausgangsstoffe, so kann der Reaktionsablauf beim erfindungsgemäßen Verfahren durch das folgende Formelschema skizziert werden:

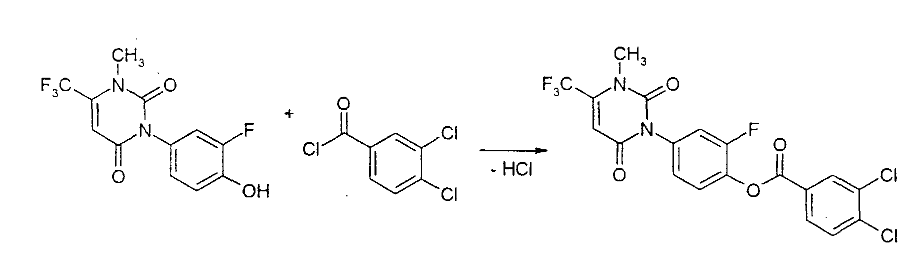

Die beim erfindungsgemäßen Verfahren zur Herstellung von Verbindungen der Formel (I) als Ausgangsstoffe zu verwendenden Hydroxyphenyluracile sind durch die Formel (II) allgemein definiert. In der Formel (II) haben n, R¹, R², R³ und R⁴ vorzugsweise bzw. insbesondere diejenigen Bedeutungen, die bereits vorstehend im Zusammenhang mit der Beschreibung der erfindungsgemäßen Verbindungen der Formel (I) vorzugsweise bzw. als insbesondere bevorzugt für n, R¹, R², R³ und R⁴ angegeben wurden.

Die Ausgangsstoffe der allgemeinen Formel (II) sind bekannt und/oder können nach an sich bekannten Verfahren hergestellt werden (vgl. EP-A-545206, JP-A-04178373).

Noch nicht aus der Literatur bekannt sind die Aminouracile der allgemeinen Formel (IIa) 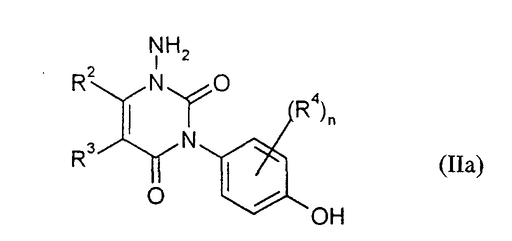 in welcher
- n, R², R³ und R⁴: die oben angegebene Bedeutung haben.

Man erhält die neuen Aminouracile der allgemeinen Formel (IIa), wenn man Uracile der allgemeinen Formel (IIb) 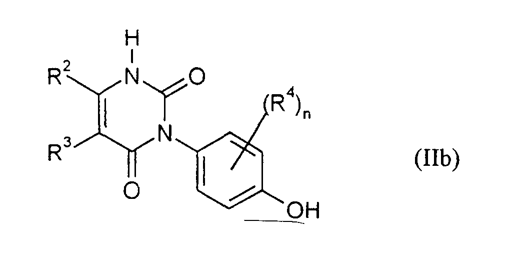 in welcher
- n, R², R³ und R⁴: die vorstehend angegebene Bedeutung haben,
mit einem elektrophilen Aminierungsmittel, wie z.B. 1-Aminooxy-2,3-dinitro-benzol, gegebenenfalls in Gegenwart eines Reaktionshilfsmittels, wie z.B. Natriumhydrogencarbonat, und gegebenenfalls in Gegenwart eines Verdünnungsmittels, wie z.B. N,N-Dimethyl-formamid, bei Temperaturen zwischen 0°C und 100°C umsetzt.

Die beim erfindungsgemäßen Verfahren weiter als Ausgangsstoffe zu verwendenden Acylierungsmittel sind durch die Formel (III) allgemein definiert. In der Formel (III) haben Q und R⁵ vorzugsweise bzw. insbesondere diejenigen Bedeutungen, die bereits vorstehend im Zusammenhang mit der Beschreibung der erfindungsgemäßen Verbindungen der Formel (I) vorzugsweise bzw. als insbesondere bevorzugt für Q und R⁵ angegeben wurden; X steht vorzugsweise für Fluor, Chlor, Brom oder die Gruppierung -O-CQ-R⁵; insbesondere für Chlor oder die Gruppierung -O-CQ-R⁵, wobei Q und R⁵ die vorstehend als bevorzugt bzw. als insbesondere bevorzugt angegebenen Bedeutungen haben.

Die Ausgangsstoffe der allgemeinen Formel (III) sind bekannte organische Synthesechemikalien.

Die beim erfindungsgemäßen Verfahren gegebenenfalls weiter als Ausgangsstoffe zu verwendenden Iso(thio)cyanate sind durch die Formel (IV) allgemein definiert. In der Formel (IV) hat Q vorzugsweise bzw. insbesondere diejenige Bedeutung, die bereits vorstehend im Zusammenhang mit der Beschreibung der erfindungsgemäßen Verbindungen der Formel (I) vorzugsweise bzw. als insbesondere bevorzugt für Q angegeben wurde; R⁶ steht vorzugsweise für gegebenenfalls durch Cyano, Halogen oder C₁-C₄-Alkoxy substituiertes Alkylamino mit 1 bis 5 Kohlenstoffatomen, für jeweils gegebenenfalls durch Cyano oder Halogen substituiertes Alkenylamino oder Alkinylamino mit jeweils bis zu 5 Kohlenstoffatomen, für jeweils gegebenenfalls durch Cyano, Halogen oder C₁-C₄-Alkyl substituiertes Cycloalkylamino oder Cycloalkylalkylamino mit 3 bis 6 Kohlenstoffatomen in den Cycloalkylgruppen und gegebenenfalls 1 bis 3 Kohlenstoffatomen im Alkylteil, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Arylamino oder Arylalkylamino mit jeweils 6 oder 10 Kohlenstoffatomen in den Arylgruppen und gegebenenfalls 1 bis 3 Kohlenstoffatomen im Alkylteil.

Die Ausgangsstoffe der allgemeinen Formel (IV) sind bekannte organische Synthesechemikalien.

Das erfindungsgemäße Verfahren zur Herstellung der Verbindungen der allgemeinen Formel (I) wird gegebenenfalls unter Verwendung eines Verdünnungsmittels durchgeführt. Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid. Soweit unter den Reaktionsbedingungen flüssige Carbonsäureanhydride, wie z.B. Acetanhydrid, als Ausgangsstoffe eingesetzt werden, können diese auch gleichzeitig im Überschuß als Verdünnungsmittel verwendet werden.

Das erfindungsgemäße Verfahren zur Herstellung der Verbindungen der allgemeinen Formel (I) wird gegebenenfalls unter Verwendung eines Reaktionshilfsmittels durchgeführt. Als Reaktionshilfsmittel für das erfindungsgemäße Verfahren kommen im allgemeinen die üblichen anorganischen oder organischen Basen oder Säureakzeptoren in Betracht. Hierzu gehören vorzugsweise Alkalimetall- oder Erdalkalimetall-acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calciumhydrid, Lithium-, Natrium-, Kalium- oder Calcium-hydroxid, Natrium- oder Kalium-- methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 20°C und 150°C. Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel gegebenenfalls in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cycloate, Cyclosulfamuron, Cycloxydim, Cyhalofop(-butyl), 2,4-D, 2,4-DB, 2,4-DP,-Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(methyl), Ethofumesate, Ethoxyfen, Ethoxysulfuron, Etobenzanid, Fenoxaprop-(-Pethyl), Flamprop(-isopropyl), Flamprop(-isopropyl-L), Flamprop(-methyl), Flazasulfuron, Fluazifop(-P-butyl), Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isouron, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

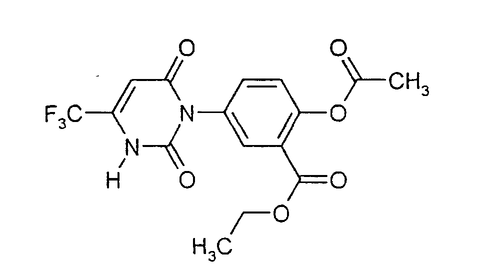

Eine Lösung von 13,8g (40 mMol) 3-(4-Hydroxy-3-ethoxycarbonyl-phenyl)-6-trifluormethyl-1,2,3,4-tetrahydro-1H-pyrimidin-2,4-dion in 100 ml Essigsäureanhydrid wird ca. 16 Stunden unter Rückfluß erhitzt. Das überschüssige Essigsäureanhydrid wird im Wasserstrahlvakuum abdestilliert, der Rückstand zweimal mit je 30 ml Diethylether verrührt und bei 50°C im Vakuum getrocknet.

Man erhält 11,5 g (74,5% der Theorie) 3-(4-Acetoxy-3-ethoxycarbonyl-phenyl)-6-trifluormethyl-1,2,3,4-tetrahydro-1H-pyrimidin-2,4-dion vom Schmelzpunkt 155°C.

### Beispiel 2

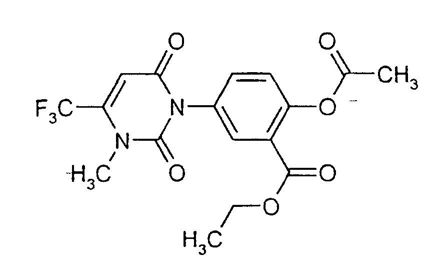

Zu einer Mischung aus 7,6 g (19,7 mMol) 3-(4-Acetoxy-3-ethoxycarbonyl-phenyl)-6-trifluormethyl-1,2,3,4-tetrahydro-1H-pyrimidin-2,4-dion und 11,0 g (80 mMol) Kaliumcarbonat in 50 ml Dimethylsulfoxid gibt man 5,0 g (40mMol) Dimethylsulfat und erwärmt 5 Stunden auf 50°C. Danach wird die Reaktionsmischung mit Wasser verdünnt und mit Dichlormethan extrahiert. Die organische Phase wird nacheinander mit gesättigter wässriger Ammoniumchlorid-Lösung und mit gesättigter wässriger Kochsalz-Lösung gewaschen, über Magnesiumsulfat getrocknet und im Wasserstrahlvakuum vom Lösungsmittel befreit. Die säulenchromatographische Trennung erfolgt über Kieselgel mit Dichlormethan / Methanol (20:1) als Laufmittel.

Man erhält 6,6 g (84% der Theorie) 3-(4-Acetoxy-3-ethoxycarbonyl-phenyl)-1-methyl-6-trifluormethyl-1,2,3,4-tetrahydro-1H-pyrimidin-2,4-dion vom Schmelzpunkt 148°C.

### Beispiel 3

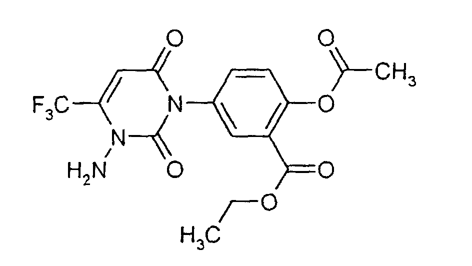

1,0 g (2,9 mMol) 3-(4-Acetoxy-3-ethoxycarbonyl-phenyl)-6-trifluormethyl-1,2,3,4-tetrahydro-1H-pyrimidin-2,4-dion werden zusammen mit 0,3 g (3,5 mMol) Natriumhydrogencarbonat und 50 ml N,N-Dimethyl-formamid eine Stunde verrührt. Dann werden 0,3 g (1,5 mMol) 1-Aminooxy-2,4-dinitro-benzol dazu gegeben und die Reaktionsmischung wird 24 Stunden bei Raumtemperatur (ca. 20°C) gerührt. Nach Zugabe von weiteren 0,3 g 1-Aminooxy-2,4-dinitro-benzol wird die Mischung weitere 24 Stunden und nach Zugabe von weiteren 0,15 g 1-Aminooxy-2,4-dinitro-benzol weitere 48 Stunden bei Raumtemperatur gerührt. Dann wird die Mischung in eine gesättigte wässrige Kochsalz-Lösung gegossen und mehrfach mit Essigsäureethylester extrahiert. Die vereinigten organischen Extraktionslösungen werden mit Wasser gewaschen, mit Natriumsulfat getrocknet und über Kieselgel filtriert. Das Filtrat wird im Wasserstrahlvakuum eingeengt, der Rückstand mit Isopropanol digeriert und das kristallin angefallene Produkt durch Absaugen isoliert.

Man erhält 0,7 g (60% der Theorie) 1-Amino-3-(4-Acetoxy-3-ethoxycarbonylphenyl)-6-trifluormethyl-1,2,3,4-tetrahydro-1H-pyrimidin-2,4-dion vom Schmelzpunkt 190°C.

Analog zu den Herstellungsbeispielen 1 bis 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

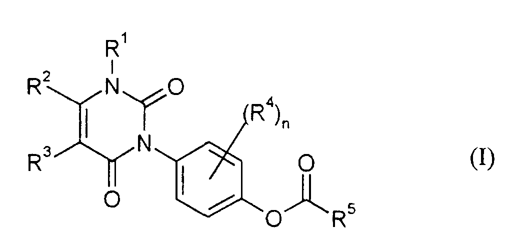

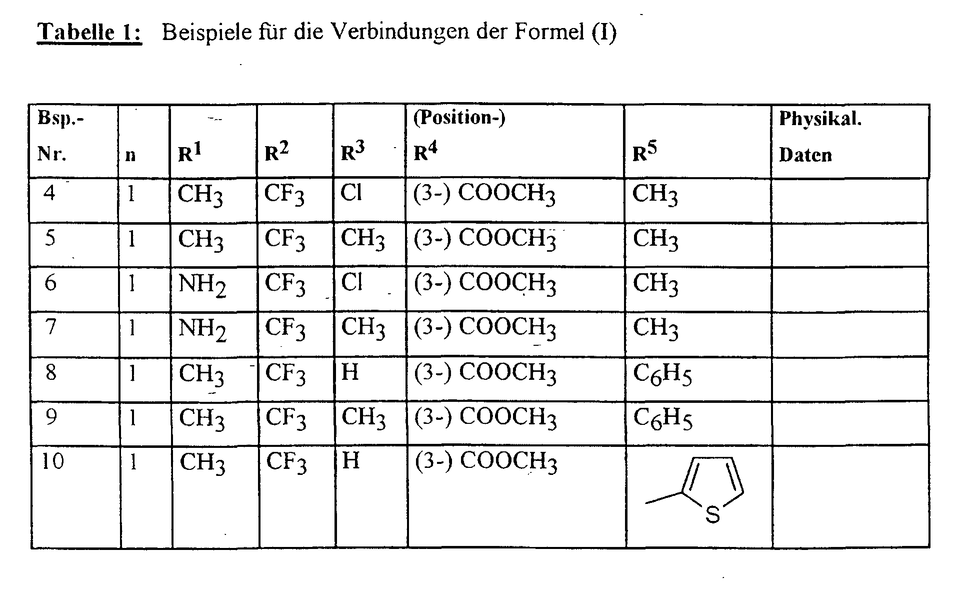

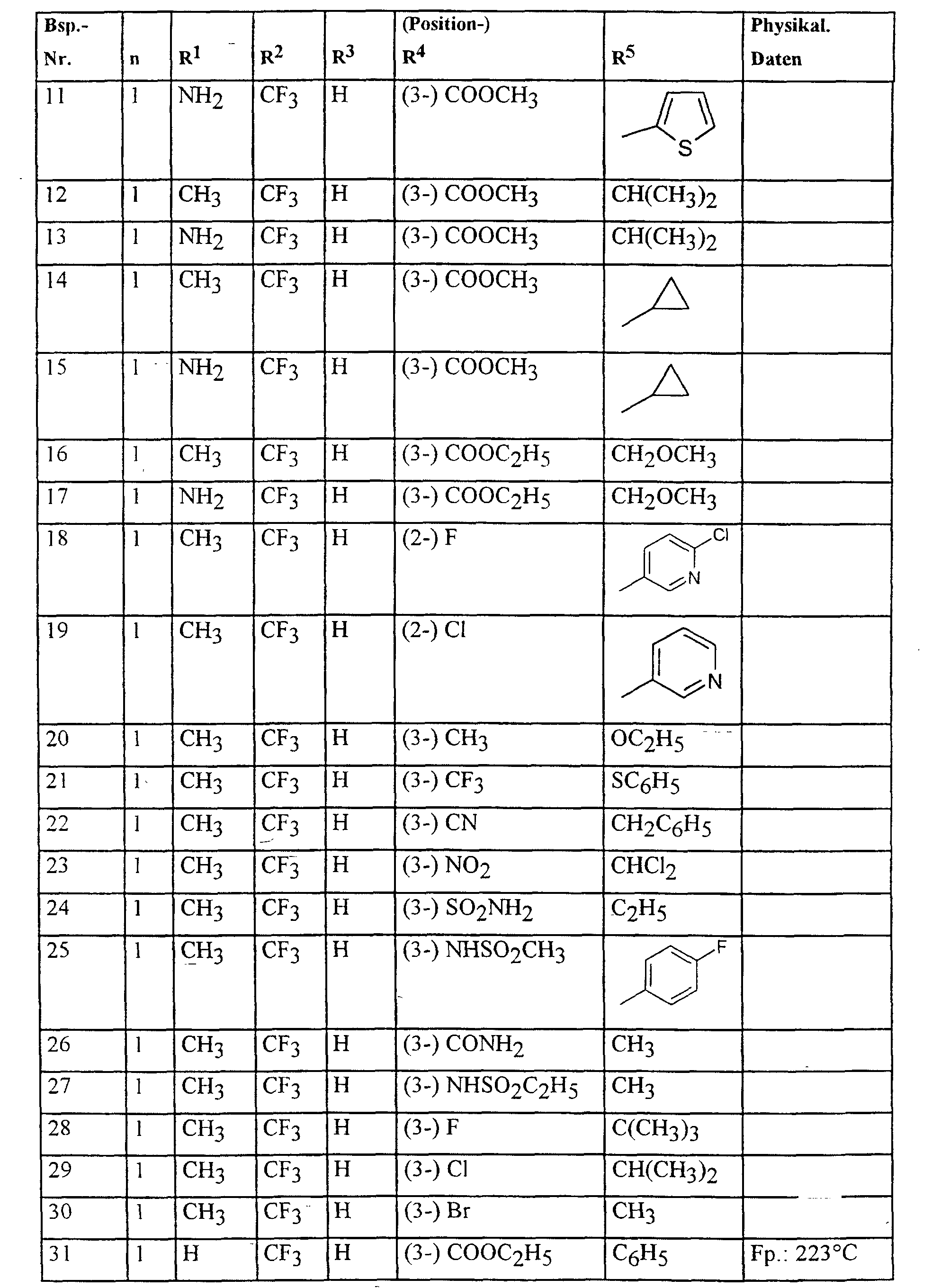

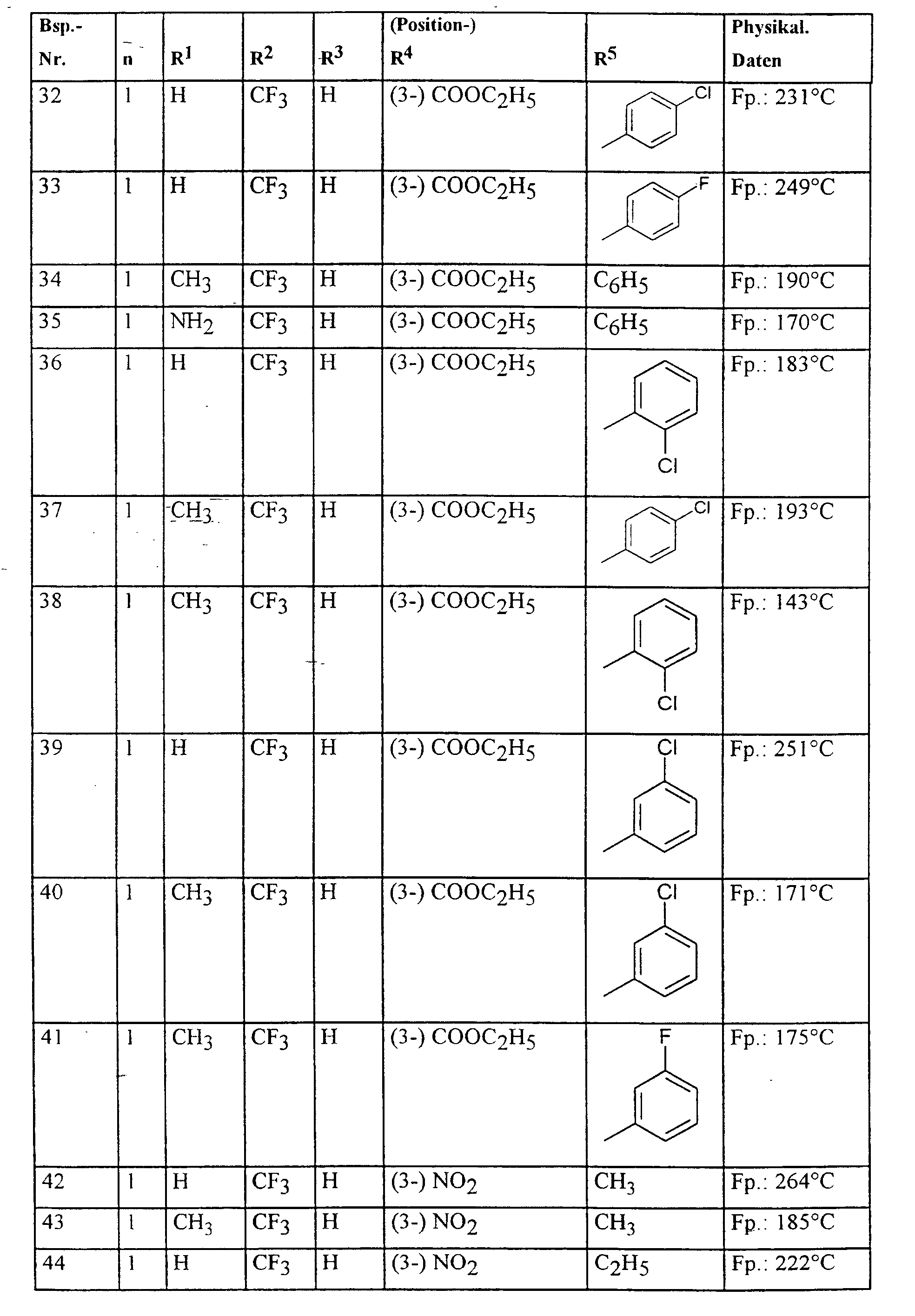

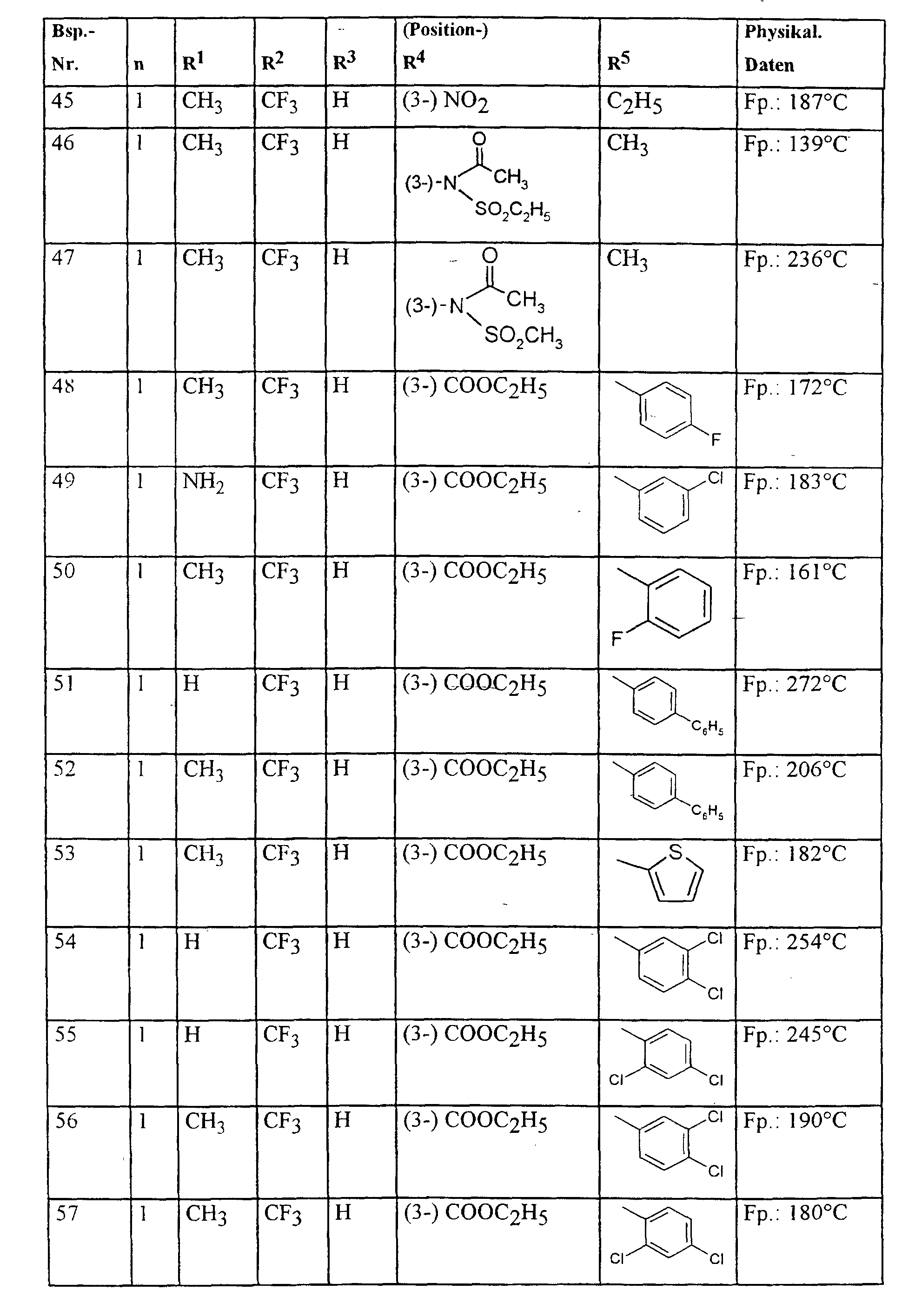

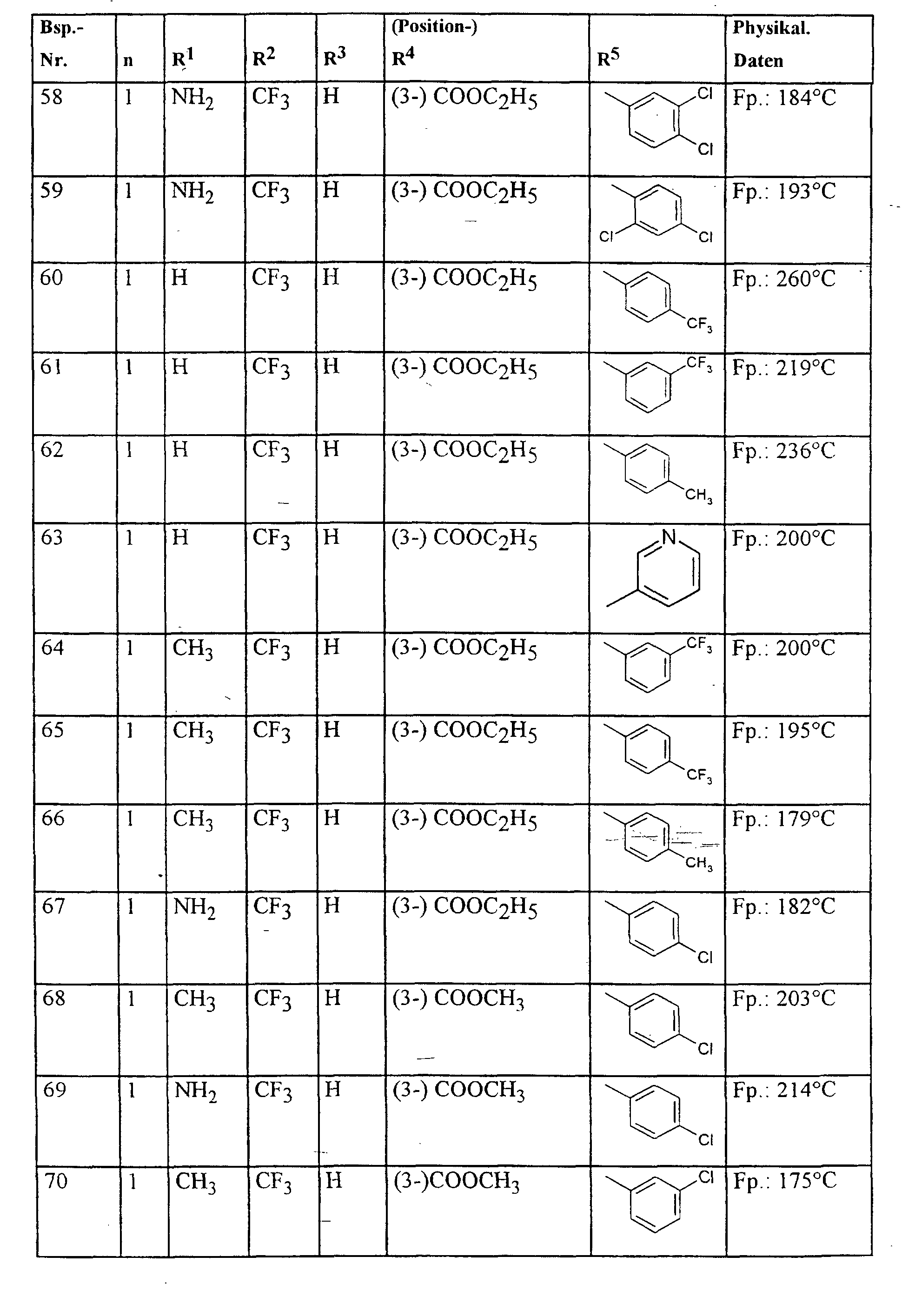

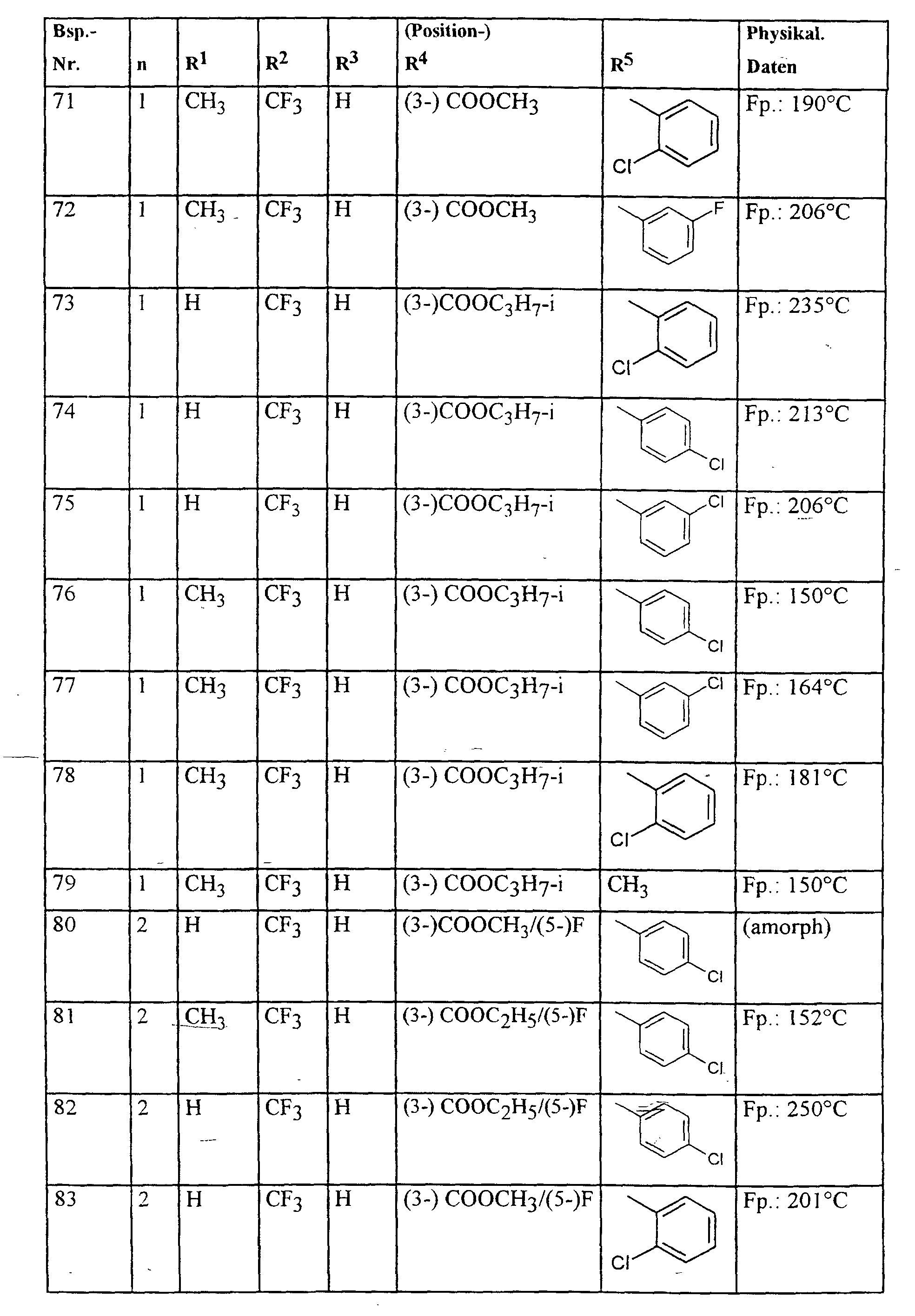

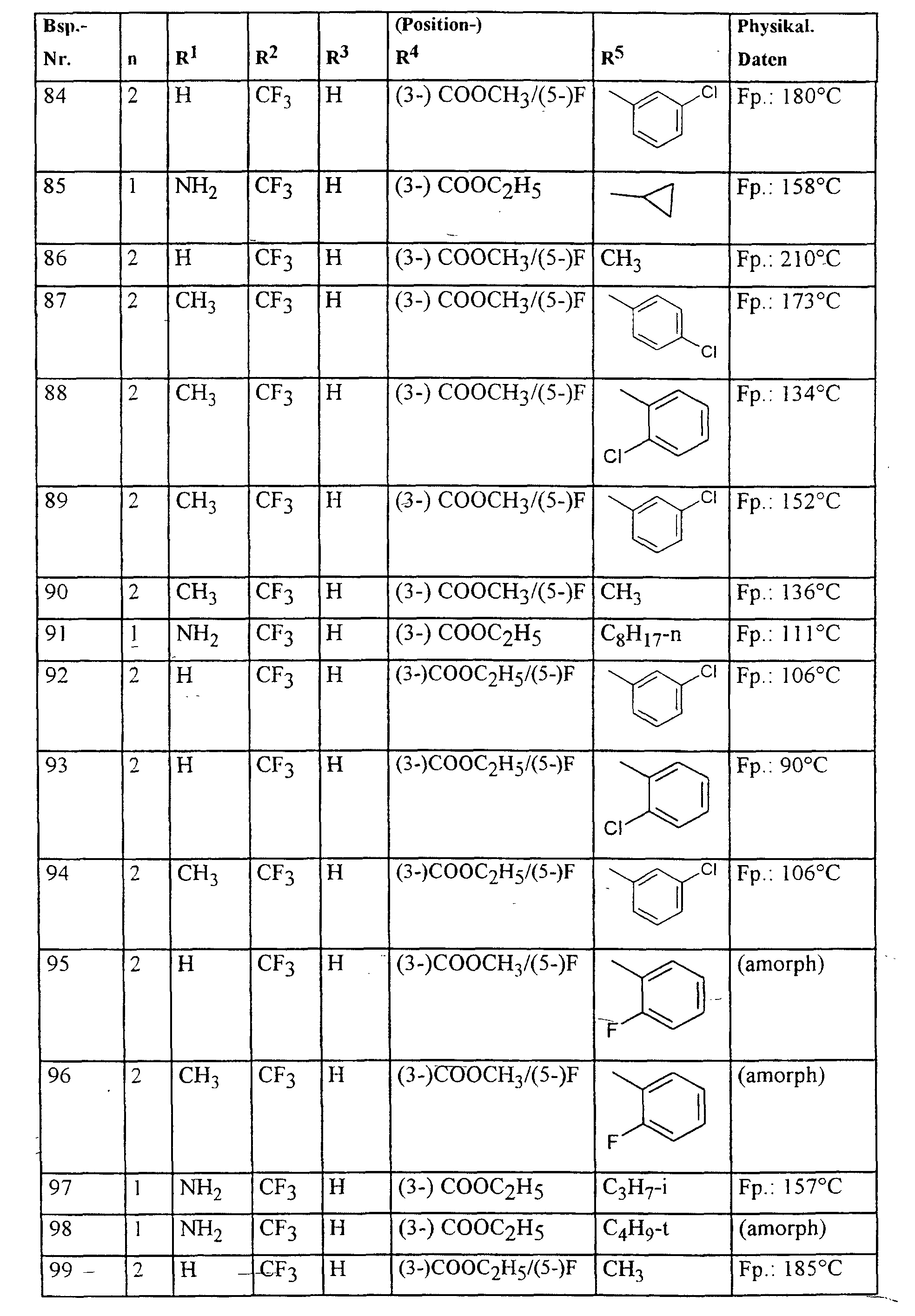

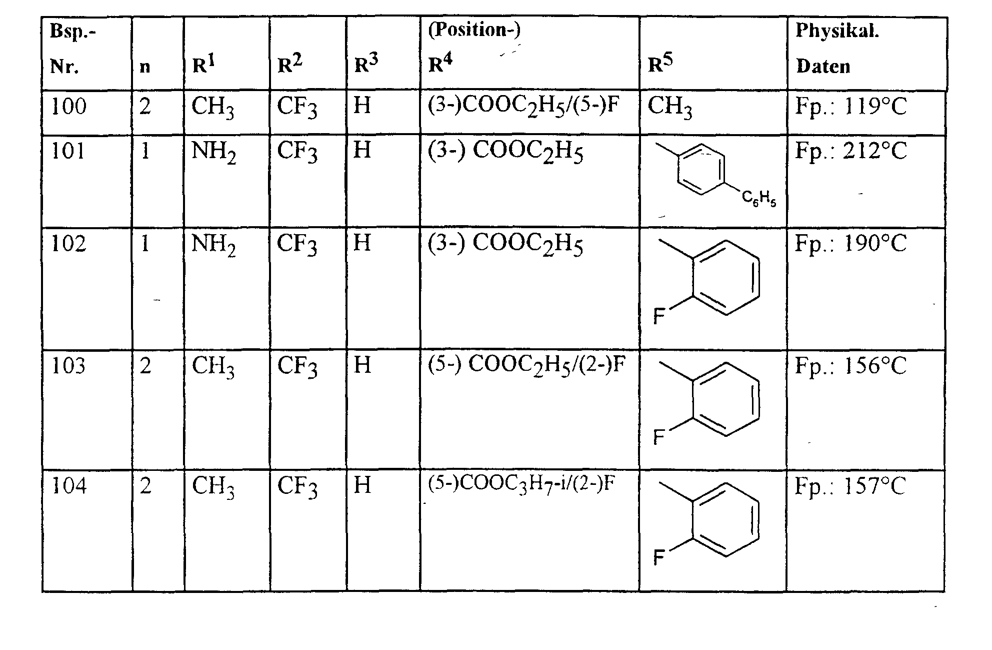

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung so gespritzt, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 2 und 3 bei teilweise guter Verträglichkeit gegenüber Kulturpflanzen, wie z.B. Baumwolle und Mais, sehr starke Wirkung gegen Unkräuter.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 2 und 3 bei teilweise guter Verträglichkeit gegenüber Kulturpflanzen, wie z.B. Raps und Weizen, sehr starke Wirkung gegen Unkräuter.

## Patentansprüche

1. Substituierte Phenyluracile der allgemeinen Formel (I), 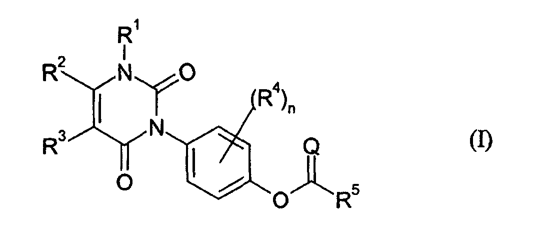 in welcher
n für die Zahlen 0, 1, 2 oder 3 steht,
Q für Sauerstoff oder Schwefel steht,
R¹ für Wasserstoff, für Amino oder für gegebenenfalls durch Cyano, Fluor, oder Chlor substituiertes Alkyl mit 1 bis 4 Kohlenstoffatomen steht,
R² für Cyano, Carboxy, Carbamoyl, Thiocarbamoyl oder für jeweils gegebenenfalls durch Cyano, Fluor oder Chlor substituiertes Alkyl oder Alkoxycarbonyl mit jeweils bis zu 4 Kohlenstoffatomen steht,
R³ für Wasserstoff, Fluor, Chlor, Brom oder Alkyl mit 1 bis 4 Kohlenstoffatomen steht,
R⁴ für Nitro, Amino, Hydroxy, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino, Alkylsulfonylamino, Bisalkylcarbonyl-amino, Bis-alkylsulfonyl-amino oder N-Alkylcarbonyl-N-alkylsulfonyl-amino mit jeweils bis zu 5 Kohlenstoffatomen in den Alkylgruppen steht, und
R⁵ für Wasserstoff, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 5 Kohlenstoffatomen, für jeweils gegebenenfalls durch Cyano oder Halogen substituiertes Alkenyl, Alkenyloxy, Alkenylamino, Alkinyl, Alkinyloxy oder Alkinylamino mit jeweils bis zu 5 Kohlenstoffatomen, für jeweils gegebenenfalls durch Cyano, Halogen oder C₁-C₄-Alkyl substituiertes Cycloalkyl, Cycloalkyloxy, Cycloalkylamino, Cycloalkylalkyl, Cycloalkylalkoxy oder Cycloalkylalkylamino mit jeweils 3 bis 6 Kohlenstoffatomen in den Cycloalkylgruppen und gegebenenfalls 1 bis 3 Kohlenstoffatomen in den Alkylteilen, für jeweils gegebenenfalls durch Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Phenyl substituiertes Aryl, Aryloxy, Arylthio, Arylamino, Arylalkyl, Arylalkoxy, Arylalkylthio oder Arylalkylamino mit jeweils 6 oder 10 Kohlenstoffatomen in den Arylgruppen und gegebenenfalls 1 bis 3 Kohlenstoffatomen in den Alkylteilen, oder für jeweils gegebenenfalls durch Cyano, Halogen, C₁-C₄-Alkyl, C₁-C₄-Halogenalkyl, C₁-C₄-Alkoxy oder C₁-C₄-Halogenalkoxy substituiertes Heterocyclyl oder Heterocyclylalkyl aus der Reihe Furyl, Tetrahydrofuryl, Furyl-C₁-C₄-alkyl, Benzofuryl, Benzofuryl-C₁-C₄-alkyl, Thienyl, Thienyl-C₁-C₄-alkyl, Benzothienyl, Benzothienyl-C₁-C₄-alkyl, Oxazolyl, Oxazolyl-C₁-C₄-alkyl, Benzoxazolyl, Benzoxazolyl-C₁-C₄-alkyl, Isoxazolyl, Isoxazolyl-C₁-C₄-alkyl, Benzisoxazolyl, Benzisoxazolyl-C₁-C₄-alkyl, Thiazoyl, Thiazolyl-C₁-C₄-alkyl, Benzthiazolyl, Benzthiazolyl-C₁-C₄-alkyl, Pyrazolyl, Pyrazolyl-C₁-C₄-alkyl, Oxadiazolyl, Oxadiazolyl-C₁-C₄-alkyl, Thiadiazolyl, Thiadiazolyl-C₁-C₄-alkyl, Pyridyl, Pyridyl-C₁-C₄-alkyl, Chinolyl, Chinolyl-C₁-C₄-alkyl, Pyrimiyl, Pyrimidyl-C₁-C₄-alkyl steht.

2. Substituierte Phenyluracile der allgemeinen Formel (I) nach Anspruch 1, **dadurch gekennzeichnet, daß**
n für die Zahlen 0, 1 oder 2 steht,
Q für Sauerstoff oder Schwefel steht,
R¹ für Wasserstoff, Amino, Methyl oder Ethyl steht,
R² für Cyano, Carboxy, Carbamoyl, Thiocarbamoyl, Methyl, Ethyl, Fluormethyl, Chlormethyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Chlorfluorethyl, Trifluorethyl, Trichlorethyl, Chlordifluorethyl, Fluordichlorethyl, Pentafluorethyl, Methoxycarbonyl oder Ethoxycarbonyl steht,
R³ für Wasserstoff, Chlor, Brom oder Methyl steht,
R⁴ für Nitro, Amino, Hydroxy, Carboxy, Cyano, Carbamoyl, Thiocarbamoyl, Sulfo, Chlorsulfonyl, Aminosulfonyl, Fluor, Chlor, Brom, oder für jeweils gegebenenfalls durch Cyano, Fluor, Chlor, Brom, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, Methylsulfonyl, Ethylsulfonyl, Methylamino, Ethylamino, n- oder i-Propylamino, n-, i-, s- oder t-Butylamino, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, Acetylamino, Propionylamino, n- oder i-Butyroylamino, Methoxycarbonylamino, Ethoxycarbonylamino, Methylsulfonylamino, Ethylsulfonylamino, n- oder i-Propylsulfonylamino, n-, i-, s- oder t-Butylsulfonylamino, Bis-methylsulfonyl-amino, Bis-ethylsulfonyl-amino, N-Acetyl-N-methylsulfonyl-amino oder N-Acetyl-N-ethylsulfonyl-amino steht, und
R⁵ für Wasserstoff, für jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, für jeweils gegebenenfalls durch Cyano, Fluor, Chlor oder Brom substituiertes Ethenyl, Propenyl, Butenyl, Propenyloxy, Butenyloxy, Propenylamino, Butenylamino, Ethinyl, Propinyl, Butinyl, Propinyloxy, Butinyloxy, Propinylamino oder Butinylamino, für jeweils gegebenenfalls durch Cyano, Fluor, Chlor, Methyl oder Ethyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylamino, Cyclobutylamino, Cyclo-pentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclopropylmethylamino, Cyclobutylmethylamino, Cyclopentylmethylamino oder Cyclohexylmethylamino, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl oder Phenyl substituiertes Phenyl, Phenoxy, Phenylthio, Phenylamino, Benzyl, Benzyloxy, Benzylthio oder Benzylamino, oder für jeweils gegebenenfalls durch Cyano, Fluor, Chlor, Brom, Methyl, Ethyl, n- oder i-Propyl, n-, i-, s- oder t-Butyl, Trifluormethyl, Methoxy, Ethoxy, n - oder i-Propoxy, Difluormethoxy oder Trifluormethoxy substituiertes Heterocyclyl oder Heterocyclylalkyl aus der Reihe Furyl, Tetrahydrofuryl, Furylmethyl, Benzofuryl, Benzofurylmethyl, Thienyl, Thienylmethyl, Benzothienyl, Benzothienylmethyl, Oxazolyl, Oxazolylmethyl, Benzoxazolyl, Benzoxazolylmethyl, Isoxazolyl, Isoxazolylmethyl, Benzisoxazolyl, Benzisoxazolylmethyl, Thiazoyl, Thiazolylmethyl, Benzthiazolyl, Benzthiazolylmethyl, Pyrazolyl, Pyrazolylmethyl, Oxadiazolyl, Oxadiazolylmethyl, Thiadiazolyl, Thiadiazolylmethyl, Pyridyl, Pyridylmethyl, Chinolyl, Chinolylmethyl, Pyrimidyl, Pyrimidylmethyl steht.

3. Verfahren zur Herstellung von substituierten Phenyluracilen der allgemeinen Formel (I) nach Anspruch 1 oder 2, **gekennzeichnet durch** Umsetzen von Hydroxyphenyluracilen der allgemeinen Formel (II) 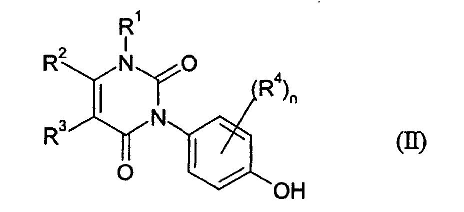 in welcher
n, R¹, R², R³ und R⁴ die in Anspruch 1 oder 2 angegebene Bedeutung haben,
mit Acylierungsmitteln der allgemeinen Formel (III) 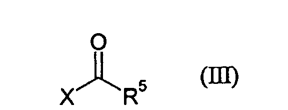 in welcher
R⁵ die in Anspruch 1 oder 2 angegebene Bedeutung hat und
X für Halogen oder die Gruppierung -O-CO-R⁵ steht
gegebenenfalls in Gegenwart eines Verdünnungsmittels und gegebenenfalls in Gegenwart eines Reaktionshilfsmittels.

4. Verfahren zur Herstellung von substituierten Phenyluracilen der allgemeinen Formel (I) nach einem der Ansprüche 1 bis 3, wobei
R⁵ für Alkylamino, Alkenylamino oder Alkinylamino mit jeweils bis zu 5 Kohlenstoffatomen, Cycloalkylamino oder Cycloalkylalkylamino mit jeweils 3 bis 6 Kohlenstoffatomen in den Cycloalkylgruppen und und gegebenenfalls 1 bis 3 Kohlenstoffatomen in den Alkylteilen, Arylamino oder Arylalkylamino mit jeweils 6 oder 10 Kohlenstoffatomen in den Arylteilen und gegebenenfalls 1 bis 3 Kohlenstoffatomen in den Alkylteilen steht,
**gekennzeichnet durch** Umsetzen von Hydroxyphenyluracilen der allgemeinen Formel (II) 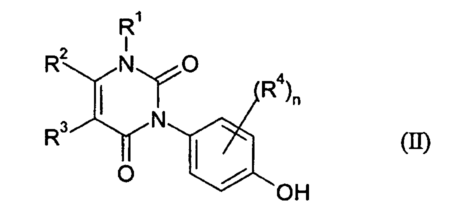 in welcher
n, R¹, R², R³ und R⁴ die in Anspruch 1 oder 2 angegebene Bedeutung haben,
mit Iso(thio)cyanaten der allgemeinen Formel (IV)
Q=C=N-R⁶ (IV)
in welcher
Q die in Anspruch 1 oder 2 angegebene Bedeutung hat und
R⁶ für gegebenenfalls **durch** Cyano, Halogen oder C₁-C₄-Alkoxy substituiertes Alkylamino mit 1 bis 5 Kohlenstoffatomen, für jeweils gegebenenfalls **durch** Cyano oder Halogen substituiertes Alkenylamino oder Alkinylamino mit jeweils bis zu 5 Kohlenstoffatomen, für jeweils gegebenenfalls **durch** Cyano, Halogen oder C₁-C₄-Alkyl substituiertes Cycloalkylamino oder Cycloalkylalkylamino mit 3 bis 6 Kohlenstoffatomen in den Cycloalkylgruppen und gegebenenfalls 1 bis 3 Kohlenstoffatomen im Alkylteil, oder für jeweils gegebenenfalls **durch** Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, Difluormethylthio, Trifluormethylthio, Methylsulfinyl, Ethylsulfmyl, Trifluormethylsulfinyl, Methylsulfonyl, Ethylsulfonyl oder Trifluormethylsulfonyl substituiertes Arylamino oder Arylalkylamino mit jeweils 6 oder 10 Kohlenstoffatomen in den Arylgruppen und gegebenenfalls 1 bis 3 Kohlenstoffatomen im Alkylteilsteht,
gegebenenfalls in Gegenwart eines Verdünnungsmittels und gegebenenfalls in Gegenwart eines Reaktionshilfsmittels.

5. Verwendung von mindestens einem substituierten Phenyluracil gemäß Anspruch 1 oder 2 zur Bekämpfung von unerwünschten Pflanzen.

6. Herbizides Mittel, **gekennzeichnet durch** den Gehalt an mindestens einem substituierten Phenyluracil gemäß Anspruch 1 oder 2.

## Claims

1. Substituted phenyluracils of the general formula (I) 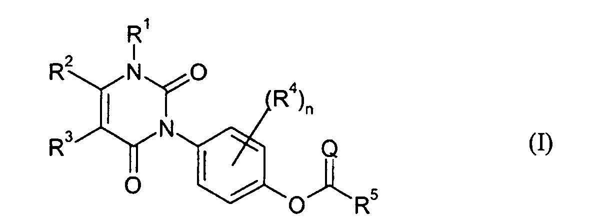 in which
n represents the numbers 0, 1, 2 or 3,
Q represents oxygen or sulphur,
R¹ represents hydrogen, represents amino or represents optionally cyano-, fluorine- or chlorine-substituted alkyl having 1 to 4 carbon atoms,
R² represents cyano, carboxyl, carbamoyl, thiocarbamoyl or represents in each case optionally cyano-, fluorine- or chlorine-substituted alkyl or alkylcarbonyl having in each case up to 4 carbon atoms,
R³ represents hydrogen, fluorine, chlorine, bromine or alkyl having 1 to 4 carbon atoms,
R⁴ represents nitro, amino, hydroxyl, carboxyl, cyano, carbamoyl, thiocarbamoyl, sulpho, chlorosulphonyl, aminosulphonyl, halogen, or represents in each case optionally cyano-, halogen- or C₁-C₄-alkoxy-substituted alkyl, alkoxy, alkylthio, alkylsulphinyl, alkylsulphonyl, alkylamino, alkylcarbonyl, alkoxycarbonyl, alkylaminocarbonyl, alkylcarbonylamino, alkoxycarbonylamino, alkylsulphonylamino, bis-alkylcarbonylamino, bis-alkylsulphonylamino or N-alkylcarbonyl-N-alkylsulphonylamino having in each case up to 5 carbon atoms in the alkyl groups, and
R⁵ represents hydrogen, represents in each case optionally cyano-, halogen- or C₁-C₄-alkoxy-substituted alkyl, alkoxy, alkylthio, alkylamino or dialkylamino having in each case 1 to 5 carbon atoms, represents in each case optionally cyano- or halogen-substituted alkenyl, alkenyloxy, alkenylamino, alkinyl, alkinyloxy or alkinylamino having in each case up to 5 carbon atoms, represents in each case optionally cyano-, halogen- or C₁-C₄-alkyl-substituted cycloalkyl, cycloalkyloxy, cycloalkylamino, cycloalkylalkyl, cycloalkylalkoxy or cycloalkylalkylamino having in each case 3 to 6 carbon atoms in the cycloalkyl groups and optionally 1 to 3 carbon atoms in the alkyl moieties, represents in each case optionally nitro-, cyano-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulphinyl-, C₁-C₄-halogenoalkylsulphinyl-, C₁-C₄-alkylsulphonyl-, C₁-C₄-halogenoalkylsulphonyl- or phenyl-substituted aryl, aryloxy, arylthio, arylamino, arylalkyl, arylalkoxy, arylalkylthio or arylalkylamino having in each case 6 or 10 carbon atoms in the aryl groups and optionally 1 to 3 carbon atoms in the alkyl moieties, or represents in each case optionally cyano-, halogen-, C₁-C₄-alkyl-, C₁-C₄-halogenoalkyl-, C₁-C₄-alkoxy- or C₁-C₄-halogenoalkoxy-substituted heterocyclyl or heterocyclylalkyl from the group consisting of furyl, tetrahydrofuryl, furyl-C₁-C₄-alkyl, benzofuryl, benzofuryl-C₁-C₄-alkyl, thienyl, thienyl-C₁-C₄-alkyl, benzothienyl, benzothienyl-C₁-C₄-alkyl, oxazolyl, oxazolyl-C₁-C₄-alkyl, benzoxazolyl, benzoxazolyl-C₁-C₄-alkyl, isoxazolyl, isoxazolyl-C₁-C₄-alkyl, benzisoxazolyl, benzisoxazolyl-C₁-C₄-alkyl, thiazolyl, thiazolyl-C₁-C₄-alkyl, benzothiazolyl, benzothiazolyl-C₁-C₄-alkyl, pyrazolyl, pyrazolyl-C₁-C₄-alkyl, oxadiazolyl, oxadiazolyl-C₁-C₄-alkyl, thiadiazolyl, thiadiazolyl-C₁-C₄-alkyl, pyridyl, pyridyl-C₁-C₄-alkyl, quinolyl, quinolyl-C₁-C₄-alkyl, pyrimidyl, pyrimidyl-C₁-C₄-alkyl.

2. Substituted phenyluracils of the general formula (I) according to Claim 1, **characterized in that**
n represents the numbers 0, 1 or 2,
Q represents oxygen or sulphur,
R¹ represents hydrogen, amino, methyl or ethyl,
R² represents cyano, carboxyl, carbamoyl, thiocarbamoyl, methyl, ethyl, fluoromethyl, chloromethyl, difluoromethyl, dichloromethyl, trifluoromethyl, trichloromethyl, chlorodifluoromethyl, fluorodichloromethyl, fluoroethyl, chloroethyl, difluoroethyl, dichloroethyl, chlorofluoroethyl, trifluoroethyl, trichloroethyl, chlorodifluoroethyl, fluorodichloroethyl, pentafluoroethyl, methoxycarbonyl or ethoxycarbonyl,
R³ represents hydrogen, chlorine, bromine or methyl,
R⁴ represents nitro, amino, hydroxyl, carboxyl, cyano, carbamoyl, thiocarbamoyl, sulpho, chlorosulphonyl, aminosulphonyl, fluorine, chlorine, bromine, or represents in each case optionally cyano-, fluorine-, chlorine-, bromine-, methoxy- or ethoxy-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, methylsulphonyl, ethylsulphonyl, methylamino, ethylamino, n- or i-propylamino, n-, i-, s- or t-butylamino, acetyl, propionyl, n- or i-butyroyl, methoxycarbonyl, ethoxycarbonyl, n- or i-propoxycarbonyl, methylaminocarbonyl, ethylaminocarbonyl, acetylamino, propionylamino, n- or i-butyroylamino, methoxycarbonylamino, ethoxycarbonylamino, methylsulphonylamino, ethylsulphonylamino, n- or i-propylsulphonylamino, n-, i-, s- or t-butylsulphonylamino, bis-methylsulphonylamino, bis-ethylsulphonylamino, N-acetyl-N-methylsulphonylamino or N-acetyl-N-ethylsulphonylamino, and
R⁵ represents hydrogen, represents in each case optionally cyano-, fluorine-, chlorine-, methoxy-, ethoxy-, n- or i-propoxy-substituted methyl, ethyl, n- or i-propyl, n-, i-, s- or t-butyl, methoxy, ethoxy, n - or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino or diethylamino, represents in each case optionally cyano-, fluorine-, chlorine- or bromine-substituted ethenyl, propenyl, butenyl, propenyloxy, butenyloxy, propenylamino, butenylamino, ethinyl, propinyl, butinyl, propinyloxy, butinyloxy, propinylamino or butinylamino, represents in each case optionally cyano-, fluorine-, chlorine-, methyl- or ethyl-substituted cyclopropyl, cyclobutyl, cyclopentyl, cyclohexyl, cyclopropyloxy, cyclobutyloxy, cyclopentyloxy, cyclohexyloxy, cyclopropylamino, cyclobutylamino, cyclo-pentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclopropylmethylamino, cyclobutylmethylamino, cyclopentylmethylamino or cyclohexylmethylamino, represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, difluoromethylthio-, trifluoromethylthio-, methylsulphinyl-, ethylsulphinyl-, trifluoromethylsulphinyl-, methylsulphonyl-, ethylsulphonyl-, trifluoromethylsulphonyl- or phenyl-substituted phenyl, phenoxy, phenylthio, phenylamino, benzyl, benzyloxy, benzylthio or benzylamino, or represents in each case optionally cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy- or trifluoromethoxy-substituted heterocyclyl or heterocyclylalkyl from the group consisting of furyl, tetrahydrofuryl, furylmethyl, benzofuryl, benzofurylmethyl, thienyl, thienylmethyl, benzothienyl, benzothienylmethyl, oxazolyl, oxazolylmethyl, benzoxazolyl, benzoxazolylmethyl, isoxazolyl, isoxazolylmethyl, benzisoxazolyl, benzisoxazolylmethyl, thiazolyl, thiazolylmethyl, benzothiazolyl, benzothiazolylmethyl, pyrazolyl, pyrazolylmethyl, oxadiazolyl, oxadiazolylmethyl, thiadiazolyl, thiadiazolylmethyl, pyridyl, pyridylmethyl, quinolyl, quinolylmethyl, pyrimidyl, pyrimidylmethyl.

3. Process for preparing substituted phenyluracils of the general formula (I) according to Claims 1 or 2, **characterized in that** hydroxyphenyluracils of the general formula (II) 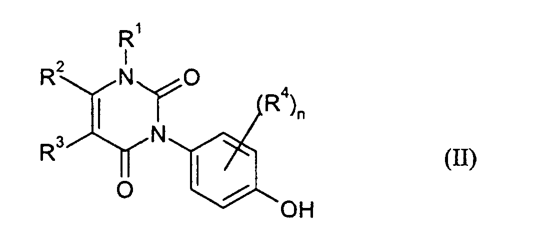 in which
n, R¹, R², R³ and R⁴ are as defined in Claims 1 or 2
are reacted with acylating agents of the general formula (III) 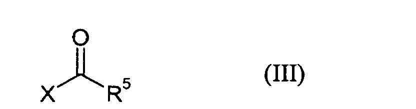 in which
R⁵ is as defined in Claims 1 or 2 and
X represents halogen or the grouping -O-CO-R⁵,
if appropriate in the presence of a diluent and if appropriate in the presence of a reaction auxiliary.

4. Process for preparing substituted phenyluracils of the general formula (I) according to any of Claims 1 to 3, where
R⁵ represents alkylamino, alkenylamino or alkinylamino having in each case up to 5 carbon atoms, cycloalkylamino or cycloalkylalkylamino having in each case 3 to 6 carbon atoms in the cycloalkyl groups and optionally 1 to 3 carbon atoms in the alkyl moieties, arylamino or arylalkylamino having in each case 6 or 10 carbon atoms in the aryl moieties and optionally 1 to 3 carbon atoms in the alkyl moieties,
**characterized in that** hydroxyphenyluracils of the general formula (II) 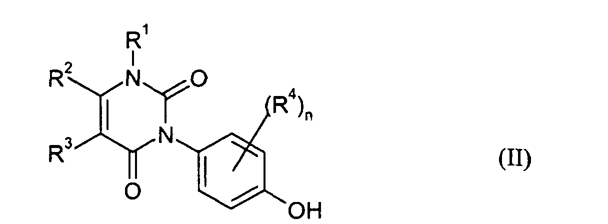 in which
n, R¹, R², R³ and R⁴ are as defined in Claims 1 or 2
are reacted with iso(thio)cyanates of the general formula (IV)
Q=C=N-R⁶ (IV)
in which
Q is as defined in Claims 1 or 2 and
R⁶ represents optionally cyano-, halogen- or C₁-C₄-alkoxy-substituted alkylamino having 1 to 5 carbon atoms, represents in each case optionally cyano- or halogen-substituted alkenylamino or alkinylamino having in each case up to 5 carbon atoms, represents in each case optionally cyano-, halogen- or C₁-C₄-alkyl-substituted cycloalkylamino or cycloalkylalkylamino having 3 to 6 carbon atoms in the cycloalkyl groups and optionally 1 to 3 carbon atoms in the alkyl moiety, or represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, difluoromethylthio-, trifluoromethylthio-, methylsulphinyl-, ethylsulphinyl-, trifluoromethylsulphinyl-, methylsulphonyl-, ethylsulphonyl- or trifluoromethylsulphonyl-substituted arylamino or arylalkylamino having in each case 6 or 10 carbon atoms in the aryl groups and optionally 1 to 3 carbon atoms in the alkyl moiety,
if appropriate in the presence of a diluent and if appropriate in the presence of a reaction auxiliary.

5. Use of at least one substituted phenyluracil according to Claims 1 or 2 for controlling undesirable plants.

6. Herbicidal composition, **characterized in that** it comprises at least one substituted phenyluracil according to Claims 1 or 2.

## Revendications

1. Phényluraciles substitués de formule générale (I) 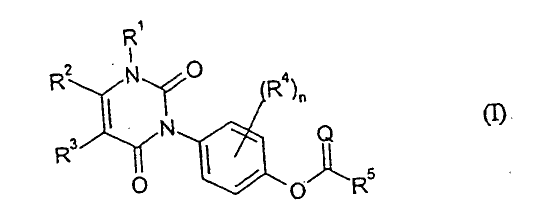 dans laquelle
n représente les nombres 0, 1, 2 ou 3,
Q est l'oxygène ou le soufre,
R¹ est l'hydrogène, un groupe amino ou un reste alkyle ayant 1 à 4 atomes de carbone éventuellement substitué par un radical cyano, du fluor ou du chlore,
R² est un groupe cyano, carboxy, carbamoyle, thiocarbamoyle ou bien un reste alkyle ou un reste alkoxycarbonyle ayant chacun jusqu'à 4 atomes de carbone et portant chacun, le cas échéant, un substituant cyano, fluoro ou chloro,
R³ est l'hydrogène, le fluor, le chlore, le brome ou un reste alkyle ayant 1 à 4 atomes de carbone,
R⁴ est un groupe nitro, amino, hydroxy, carboxy, cyano, carbamoyle, thiocarbamoyle, sulfo, chlorosulfonyle, aminosulfonyle, halogéno, ou un reste alkyle, alkoxy, alkylthio, alkylsulfinyle, alkylsulfonyle, alkylamino, alkylcarbonyle, alkoxycarbonyle, alkylaminocarbonyle, alkylcarbonylamino, alkoxycarbonylamino, alkylsulfonylamino, bis-alkylcarbonylamino, bis-alkylsulfonylamino ou N-alkylcarbonyl-N-alkylsulfonylamino ayant chacun jusqu'à 5 atomes de carbone dans les groupes alkyle et portant chacun, le cas échéant un substituant cyano, halogéno ou alkoxy en C₁ à C₄, et
R⁵ représente l'hydrogène, un reste alkyle, alkoxy, alkylthio, alkylamino ou dialkylamino ayant dans chaque cas 1 à 5 atomes de carbone et portant éventuellement un substituant cyano, halogéno ou alkoxy en C₁ à C₄, un reste alcényle, alcényloxy, alcénylamino, alcynyle, alcynyloxy ou alcynylamino ayant dans chaque cas jusqu'à 5 atomes de carbone et portant éventuellement un substituant cyano ou halogéno, un reste cycloalkyle, cycloalkyloxy, cycloalkylamino, cycloalkylalkyle, cycloalkylalkoxy. ou cycloalkylalkylamino, ayant dans chaque cas 3 à 6 atomes de carbone dans les groupes cyclalkyle et le cas échéant, 1 à 3 atomes de carbone dans les parties alkyle et portant chacun éventuellement un substituant cyano, halogéno ou alkyle en C₁ à C₄, un reste aryle, aryloxy, arylthio, arylamino, arylalkyle, arylalkoxy, arylalkylthio ou arylalkylamino ayant dans chaque cas 6 ou 10 atomes de carbone des les groupes aryle et, le cas échéant 1 à 3 atomes de carbone dans les parties alkyle et portant chacun, le cas échéant, un substituant nitro, cyano, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄ ou phényle, ou bien un reste hétérocyclyle ou hétérocyclylalkyle de la série furyle, tétrahydrofuryle, furyl(alkyle en C₁ à C₄), benzofuryle, benzofuryl(alkyle en C₁ à C₄), thiényle, thiényl-(alkyle en C₁ à C₄), benzothiényle, benzothiényl-(alkyle en C₁ à C₄), oxazolyle, oxazolyl-(alkyle en C₁ à C₄), benzoxazolyle, benzoxazolyl-(alkyle en C₁ à C₄), isoxazolyle, isoxazolyl-(alkyle en C₁ à C₄), benzisoxazolyle, benzisoxazolyl-(alkyle en C₁ à C₄), thiazoyle, thiazolyl-(alkyle en C₁ à C₄), benzthiazolyle, benzthiazolyl-(alkyle en C₁ à C₄), pyrazolyle, pyrazolyl-(alkyle en C₁ à C₄), oxadiazolyle, oxadiazolyl-(alkyle en C₁ à C₄), thiadiazolyle, thiadiazolyl-(alkyle en C₁ à C₄), pyridyle, pyridyl-(alkyle en C₁ à C₄), quinolyle, quinolyl-(alkyle en C₁ à C₄), pyrimidyle, pyrimidyl-(alkyle en C₁ à C₄) portant chacun, le cas échéant, un substituant cyano, halogéno, alkyle en C₁ à C₄, halogénalkyle en C₁ à C₄, alkoxy en C₁ à C₄ ou halogénalkoxy en C₁ à C₄.

2. Phényluraciles substitués de formule générale (I) suivant la revendication 1, **caractérisés en ce que** :
n représente les nombres 0, 1 ou 2,
Q est l'oxygène ou le soufre,
R¹ est l'hydrogène, un groupe amino, méthyle ou éthyle,
R² est un groupe cyano, carboxy, carbamoyle, thiocarbamoyle, un reste méthyle, éthyle, fluorométhyle, chlorométhyle, difluorométhyle, dichlorométhyle, trifluorométhyle, trichlorométhyle, chlorodifluorométhyle, fluorodichlorométhyle, fluoréthyle, chloréthyle, difluoréthyle, dichloréthyle, chlorofluoréthyle, trifluoréthyle, trichloréthyle, chlorodifluoréthyle, fluorodichloréthyle, pentafluoréthyle, méthoxycarbonyle ou éthoxycarbonyle,
R³ est l'hydrogène, le chlore, le brome ou un groupe méthyle,
R⁴ est un groupe nitro, amino, hydroxy, carboxy, cyano, carbamoyle, thiocarbamoyle, sulfo, chlorosulfonyle, aminosulfonyle, le fluor, le chlore, le brome ou un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylsulfinyle, éthylsulfinyle, méthylsulfonyle, éthylsulfonyle, méthylamino, éthylamino, n-propylamino, iso-propylamino, n-butylamino, isobutylamino, sec.-butylamino, tertiobutylamino, acétyle, propionyle, n-butyroyle, isobutyroyle, méthoxycarbonyle, éthoxycarbonyle, n-propoxycarbonyle, isopropoxycarbonyle, méthylaminocarbonyle, éthylaminocarbonyle, acétylamino, propionylamino, n-butyroylamino, isobutyroylamino, méthoxycarbonylamino, éthoxycarbonylamino, méthylsulfonylamino, éthylsulfonylamino, n-propyl-sulfonylamino, isopropylsulfonylamino, n-butyl-sulfonylamino, isobutylsulfonylamino, sec.-butyl-sulfonylamino, tertiobutylsulfonylamino, bis-méthyl-sulfonylamino, bis-éthylsulfonylamino, N-acétyl-N-méthylsulfonylamino ou N-acétyl-N-éthylsulfonylamino portant chacun, le cas échéant un substituant cyano, fluoro, chloro, bromo, méthoxy ou éthoxy, et
R⁵ représente l'hydrogène, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylamino, éthylamino, n-propylamino, isopropylamino, n-butylamino, isobutylamino, sec.-butylamino, tertiobutylamino, diméthylamino ou diéthylamino portant chacun éventuellement un substituant cyano, fluoro, chloro, méthoxy, éthoxy, n-propoxy ou isopropoxy, un reste éthényle, propényle, butényle, propényloxy, butényloxy, propénylamino, buténylamino, éthynyle, propynyle, butynyle, propynyloxy, butynyloxy, propynylamino ou butynylamino portant chacun, le cas échéant, un substituant cyano, fluoro, chloro ou bromo, un reste cyclopropyle, cyclobutyle, cyclopentyle, cyclohexyle, cyclopropyloxy, cyclobutyloxy, cyclopentyloxy, cyclohexyloxy, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclopropylméthylamino, cyclobutylméthylamino, cyclo-pentylméthylamino ou cyclohexylméthylamino portant chacun, le cas échéant, un substituant cyano, fluoro, chloro, méthyle ou éthyle, un reste phényle, phénoxy, phénylthio, phénylamino, benzyle, benzyloxy, benzylthio ou benzylamino portant chacun, le cas échéant, un substituant nitro, cyano, fluoro, chloro, bromo, méthyl, éthyle, n-propyle, isopropyle, n-butyle, isobutyle, sec.-butyle, tertiobutyle, trifluorométhyle, méthoxy, éthoxy, n-propoxy, isopropoxy, difluorométhoxy, trifluorométhoxy, méthylthio, éthylthio, difluorométhylthio, trifluorométhylthio, méthylsulfinyle, éthylsulfinyle, trifluorométhylsulfinyle, méthylsulfonyle, éthylsulfonyle, trifluorométhylsulfonyle ou phényle, ou bien un reste hétérocyclyle ou hétérocyclylalkyle de la série furyle, tétrahydrofuryle, furylméthyle, benzofuryle, benzofurylméthyle, thiényle, thiénylméthyle, benzothiényle, benzothiénylméthyle, oxazolyle, oxazolylméthyle, benzoxazolyle, benzoxazolylméthyle, isoxazolyle, isoxazolylméthyle, benzisoxazolyle, benzisoxazolylméthyle, thiazoyle, thiazolylméthyle, benzthiazolyle, benzthiazolylméthyle, pyrazolyle, pyrazolylméthyle, oxadiazolyle, oxadiazolylméthyle, thiadiazolyle, thiadiazolylméthyle, pyridyle, pyridylméthyle, quinolyle, quinolylméthyle, pyrimidyle, pyrimidylméthyle portant chacun le cas échéant, un substituant cyano, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, difluorométhoxy ou trifluorométhoxy.

3. Procédé de production de phényluraciles substitués de formule générale (I) suivant la revendication 1 ou 2, **caractérisé par** la réaction d'hydroxyphényluraciles de formule générale (II) 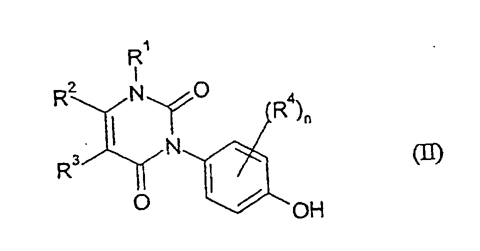 dans laquelle
n, R¹, R², R³ et R⁴ ont la définition indiquée dans la revendication 1 ou 2,
avec des agents d'acylation de formule générale (III) 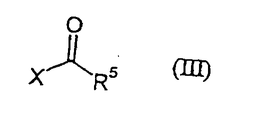 dans laquelle
R⁵ a la définition indiquée dans la revendication 1 ou 2 et
X est un halogène ou le groupement -O-CO-R⁵
le cas échéant en présence d'un diluant et en présence éventuelle d'un auxiliaire de réaction.

4. Procédé de production de phényluraciles substitués de formule générale (I) suivant l'une des revendications 1 à 3, formule dans laquelle
R⁵ est un reste alkylamino, alcénylamino ou alcynylamino ayant dans chaque cas jusqu'à 5 atomes de carbone, un reste cycloalkylamino ou cycloalkylalkylamino ayant dans chaque cas 3 à 6 atomes de carbone dans les groupes cycloalkyle et, le cas échéant, 1 à 3 atomes de carbone dans les parties alkyle, un reste arylamino ou arylalkylamino ayant dans chaque cas 6 ou 10 atomes de carbone dans les parties aryle et, le cas échéant 1 à 3 atomes de carbone dans les parties alkyle,
**caractérisé par** la réaction d'hydroxyphényluraciles de formule générale (II) 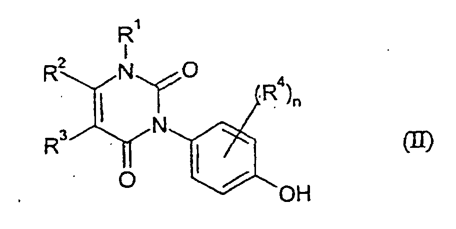 dans laquelle
n, R¹, R², R³ et R⁴ ont la définition indiquée dans la revendication 1 ou 2,
avec des iso-(thio)-cyanates de formule (IV)
Q=C=N-R⁶ (IV)
dans laquelle
Q a la définition indiquée dans la revendication 1 ou 2 et
R⁶ est un reste alkylamino ayant 1 à 5 atomes de carbone éventuellement substitué par un radical cyano, halogéno ou alkoxy en C₁ à C₄, un reste alcénylamino ou alcynylamino ayant dans chaque cas jusqu'à 5 atomes de carbone et portant éventuellement un substituant cyano ou halogéno, un reste cycloalkylamino ou cycloalkylalkylamino ayant 3 à 6 atomes de carbone dans les groupes cycloalkyle et, le cas échéant, 1 à 3 atomes de carbone dans la partie alkyle et portant chacun éventuellement un substituant cyano, halogéno ou alkyle en C₁ à C₄, ou bien un reste arylamino ou arylalkylamino ayant dans chaque cas 6 ou 10 atomes de carbone dans les groupes aryle et, le cas échéant, 1 à 3 atomes de carbone dans la partie alkyle et portant chacun éventuellement un substituant nitro, cyano, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, difluorométhoxy, trifluorométhoxy, méthylthio, éthylthio, difluorométhylthio, trifluorométhylthio, méthylsulfinyle, éthylsulfinyle, trifluorométhylsulfinyle, méthylsulfonyle, éthylsulfonyle ou trifluorométhylsulfonyle, le cas échéant en présence d'un diluant et en présence éventuelle d'un auxiliaire de réaction.

5. Utilisation d'au moins un phényluracile substitué suivant la revendication ou 2 pour combattre des plantes indésirables.

6. Composition herbicide, **caractérisée par** une teneur en au moins un phényluracile substitué suivant la revendication 1 ou 2.
